(19) Europäisches Patentamt

European Patent Office

Office européen des brevets

(11) **EP 2 412 390 A2**

(12) **EUROPEAN PATENT APPLICATION**

(43) Date of publication:
**01.02.2012 Bulletin 2012/05**

(51) Int Cl.:
***A61L 15/58*** *(2006.01)*

(21) Application number: **11175477.6**

(22) Date of filing: **27.07.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.07.2010 JP 2010169751**

(71) Applicant: **Nitto Denko Corporation**
**Ibaraki-shi,**
**Osaka 567-8680 (JP)**

(72) Inventors:
• **Hanatani, Akinori**
**Ibaraki-shi, Osaka 567-8680 (JP)**

• **Sakamoto, Sachiko**
**Ibaraki-shi, Osaka 567-8680 (JP)**
• **Okazaki, Arimichi**
**Ibaraki-shi, Osaka 567-8680 (JP)**
• **Akemi, Hitoshi**
**Ibaraki-shi, Osaka 567-8680 (JP)**

(74) Representative: **Grünecker, Kinkeldey,**
**Stockmair & Schwanhäusser**
**Anwaltssozietät**
**Leopoldstrasse 4**
**80802 München (DE)**

(54) **Adhesive patch and patch preparation**

(57)    The present invention provides an adhesive patch including a backing and a pressure-sensitive adhesive layer formed on at least one surface of the backing, in which (a) a surface of the pressure-sensitive adhesive layer has a logarithmic decrement, as determined by the rigid-pendulum free-damped oscillation method, in the range of 0.03 to 0.35, and (b) the pressure-sensitive adhesive layer has a maximum shear displacement in the range of 18 μm to 1,000 μm. The present invention further provides a patch preparation containing a drug. The adhesive patch and the patch preparation according to the present invention are reduced in skin irritation during wear.

**Description**

FIELD OF THE INVENTION

The present invention relates to an adhesive patch and a patch preparation which are reduced in skin irritation during wear.

BACKGROUND OF THE INVENTION

Adhesive patches and patch preparations, which are intended to be used for covering and protecting the skin, percutaneously administering drugs, etc., are required to have sufficient adhesiveness to the skin and be reduced in skin irritation. Skin irritations are roughly divided into irritation during wear and irritation at the time of peeling.
The skin irritation during wear includes chemical irritation, physical irritation, irritation due to occlusion, etc. It has been known that the chemical irritation can be relieved by changing a composition of the pressure-sensitive adhesive layer to another composition which is less irritative. It has also been known that the irritation due to occlusion can be relieved, for example, by enhancing the stretchability and moisture permeability of the adhesive patch or patch preparation as a whole. The stretchability and the moisture permeability considerably depend on the backing. However, in patch preparations intended to be used for percutaneous administration of drugs, for example, there are cases where the composition of the pressure-sensitive adhesive layer and the backing exert a considerable influence on drug penetration through the skin, and this may impose limitations on the selection of a pressure-sensitive adhesive layer composition and a backing from the standpoint of making the adhesive patch or patch preparation perform the function thereof.
Meanwhile, it has hitherto been attempted to relieve skin irritation at the time of peeling. Skin irritation at the time of peeling is considerably attributable to physical irritation due to stratum corneum separation, etc. Patent documents, for example, JP-A-5-65460, JP-A-5-139960, JP-A-6-319792 and JP-A-6-343685 include a description that this kind of irritation can be relieved mainly by regulating the material properties of the pressure-sensitive adhesive layer. However, none has been known about how the material properties of a pressure-sensitive adhesive layer are regulated in order to relieve the physical skin irritation which occurs when an adhesive patch or patch preparation is worn.

SUMMARY OF THE INVENTION

An object of the invention is to provide an adhesive patch and a patch preparation which are reduced in the skin irritation that occurs during wear.
Investigations were diligently made in order to overcome the problem. As a result, it has surprisingly been found that the skin irritation which occurs during wear of an adhesive patch or patch preparation can be relieved by regulating (a) the logarithmic decrement of the surface of the pressure-sensitive adhesive layer, which is determined by the rigid-pendulum free-damped oscillation method, and (b) the maximum shear displacement of the pressure-sensitive adhesive layer which occurs when a shear stress is imposed on the pressure-sensitive adhesive layer. The invention has been thus achieved. The invention relates to the following items.
1. An adhesive patch comprising a backing and a pressure-sensitive adhesive layer formed on at least one surface of the backing,
wherein (a) a surface of the pressure-sensitive adhesive layer has a logarithmic decrement, as determined by the rigid-pendulum free-damped oscillation method, in the range of 0.03 to 0.35, and
(b) the pressure-sensitive adhesive layer has a maximum shear displacement in the range of 18 $\mu$m to 1,000 $\mu$m.
2. The adhesive patch according to item 1, wherein the pressure-sensitive adhesive layer, upon release from the shear stress, has a percentage recovery from the shear displacement of 85% or higher.
3. The adhesive patch according to item 1, wherein the pressure-sensitive adhesive layer is a crosslinked pressure-sensitive adhesive layer comprising a polymer and an organic liquid ingredient.
4. The adhesive patch according to item 2, wherein the pressure-sensitive adhesive layer is a crosslinked pressure-sensitive adhesive layer comprising a polymer and an organic liquid ingredient.
5. The adhesive patch according to item 3, wherein the pressure-sensitive adhesive layer has been crosslinked with an external crosslinking agent.
6. The adhesive patch according to item 4, wherein the pressure-sensitive adhesive layer has been crosslinked with an external crosslinking agent.
7. A patch preparation, which is the adhesive patch according to any one of items 1 to 6, wherein the pressure-sensitive adhesive layer further contains a drug.

According to the invention, the skin irritation which occurs during wear of an adhesive patch or patch preparation can be relieved by regulating (a) the logarithmic decrement of the surface of the pressure-sensitive adhesive layer. Furthermore, by regulating (b) the maximum shear displacement of the pressure-sensitive adhesive layer, not only skin irritation during wear, in particular, skin irritation in parts of the body which are frequently bent and stretched, can be relieved but also the adhesive patch or patch preparation can be inhibited from partly or wholly peeling off the skin. Moreover, in a preferred embodiment of the invention, the pressure-sensitive adhesive layer has a specific value of percentage recovery from shear displacement. As a result, skin irritation during wear and adhesion to the skin in parts where the skin is frequently stretched and contracted can be further relieved or improved.

DETAILED DESCRIPTION OF THE INVENTION

Embodiments of the invention will be described below in detail. In the following description, the term "adhesive patch" means a conception which includes both an adhesive patch to be used as a medical or hygienic material for application to the skin and a patch preparation in which the pressure-sensitive adhesive layer contains a drug.

The skin of a human being is stretched and contracted when the human being moves, and the stretching and contraction render the movements smooth. Skin stretching and contraction occur not only in joints including the elbows and the knees but also in parts which relatively less move, such as the upper arms and the chest. Consequently, during the period when an adhesive patch is worn on the skin, the underlying skin part is prevented from stretching and contracting, and the resultant stress is causative of physical irritation.

According to the invention, an adhesive patch including a backing and a pressure-sensitive adhesive layer formed on at least one surface of the backing is configured while regulating the logarithmic decrement of the surface of the pressure-sensitive adhesive layer and the maximum shear displacement of the pressure-sensitive adhesive layer at the time where a shear stress is imposed thereon. As a result, the skin irritation which occurs during the period when the adhesive patch is worn on the skin, i.e., the physical skin irritation attributable to the stress that generates during the period when the adhesive patch is worn on the skin, is relieved. Specifically, in the adhesive patch of the invention, (a) the surface of the pressure-sensitive adhesive layer has a logarithmic decrement, as determined by the rigid-pendulum free-damped oscillation method, in the range of 0.03 to 0.35, and (b) the pressure-sensitive adhesive layer, when a shear stress is imposed thereon, has a maximum shear displacement in the range of 18 $\mu$m to 1,000 $\mu$m.

<(a) Logarithmic Decrement of Surface of the Pressure-Sensitive Adhesive Layer>
In case where the surface of a pressure-sensitive adhesive layer has a large value of logarithmic decrement, a considerable stress diminution occurs at the interface between the skin and the pressure-sensitive adhesive layer and this decrease in stress causes skin irritation. Consequently, skin irritation can be relieved by reducing the logarithmic decrement of the surface of a pressure-sensitive adhesive layer.
The surface of the pressure-sensitive adhesive layer of the adhesive patch according to the invention is required to have a logarithmic decrement in the range of 0.03 to 0.35 when examined at a sample temperature of 23°C. Furthermore, it is preferable that, at any sample temperature within 23°C to 40°C, the logarithmic decrement of the pressure-sensitive adhesive layer surface is in the range of 0.03 to 0.35, more preferably in the range of 0.05 to 0.30.
When the surface of the pressure-sensitive adhesive layer of the adhesive patch has a logarithmic decrement in the range of 0.03 to 0.35, skin irritation during wear is satisfactorily relieved. In case where the logarithmic decrement of the pressure-sensitive adhesive layer surface exceeds 0.35, a large diminution in stress occurs on the skin surface and, hence, skin irritation such as, for example, a pain or uncomfortable feeling is apt to occur during wear. On the other hand, in case where the logarithmic decrement of the pressure-sensitive adhesive layer surface is less than 0.03, the pressure-sensitive adhesive layer has too low viscosity and there are cases where sufficient adhesion to the skin is not obtained.

In the invention, the logarithmic decrement of the surface of a pressure-sensitive adhesive layer is determined by the rigid-pendulum free-damped oscillation method, and is measured with a rigid-pendulum type viscoelastometer in common use. This rigid-pendulum type viscoelastometer is an apparatus based on a measurement principle according to the rigid-pendulum free-damped oscillation method as provided for in ISO (International Standard Organization) 1522. Examples thereof include RPT-3000W, manufactured by A & D Company, Ltd. With respect to the adhesive patch of the invention, a measurement is made on samples having a width of 10 mm using a cylinder type brass edge having a diameter of 4 mm and a rigid pendulum having a moment of inertia of $6 \times 10^2$ g·cm$^2$ and a mass of 13 g.

The logarithmic decrement of the surface of a pressure-sensitive adhesive layer is affected by the kinds and contents of ingredients contained in the pressure-sensitive adhesive layer. The logarithmic decrement thereof depends largely on the kind and molecular weight of the polymer constituting the pressure-sensitive adhesive layer. Consequently, for regulating the logarithmic decrement of the surface of a pressure-sensitive adhesive layer to a value within that range, it is necessary to take account of the kind and molecular weight of the polymer to be used for forming the pressure-sensitive adhesive layer and the content of the polymer in the pressure-sensitive adhesive layer. From the standpoint of accomplishing the object of the invention, the polymer to be contained in the pressure-sensitive adhesive layer preferably is an acrylic polymer or a rubber type polymer, and especially preferably is an acrylic polymer, as will be described later. The absolute molecular weight of the polymer is preferably $1 \times 10^5$ to $6 \times 10^6$ (value determined with a gel permeation chromatograph/multi-angle laser light scattering detector; the same applies hereinafter), more preferably $3 \times 10^5$ to $5 \times 10^6$, even more preferably $1 \times 10^6$ to $4 \times 10^6$. There is a tendency that the higher the absolute molecular weight of the polymer is, the lower the logarithmic decrement of the pressure-sensitive adhesive layer surface is.

The content of the polymer in the pressure-sensitive adhesive layer is preferably 30% by weight to 95% by weight, more preferably 30% by weight to 90% by weight, although the content thereof depends on the kind and molecular weight of the polymer. There is a tendency that the higher the content of the polymer is, the lower the logarithmic decrement of the pressure-sensitive adhesive layer surface is.

Crosslinking a pressure-sensitive adhesive layer also changes the logarithmic decrement of the surface of the pressure-sensitive adhesive layer. From the standpoint of regulating the logarithmic decrement of the surface of a pressure-sensitive adhesive layer to a value within that range, it is preferred to crosslink the pressure-sensitive adhesive layer. Crosslinking a pressure-sensitive adhesive layer tends to result in a decrease in the logarithmic decrement of the surface of the pressure-sensitive adhesive layer. Techniques for crosslinking, etc. will be described later.

In patch preparations intended to be used for percutaneous administration of drugs, since there are cases where the composition constituting the pressure-sensitive adhesive layer and the compositional ratio thereof affect drug penetration through the skin, limitations may be imposed on the regulation of the logarithmic decrement of the pressure-sensitive adhesive layer surface. However, the crosslinking of the pressure-sensitive adhesive layer exerts a relatively limited influence on drug penetration through the skin. Consequently, the logarithmic decrement of the surface of the pressure-sensitive adhesive layer can be regulated by setting conditions for crosslinking a pressure-sensitive adhesive layer while avoiding drug deterioration.

The degree of freedom of selection of a pressure-sensitive adhesive layer thickness and a backing can be heightened by regulating the logarithmic decrement of the surface of the pressure-sensitive adhesive layer. As a result, it is possible to regulate drug penetration through the skin by regulating the thickness of the pressure-sensitive adhesive layer or the backing.

<(b) Maximum Shear Displacement of the Pressure-Sensitive Adhesive Layer>

The stress remaining after a diminution which occurred at the interface between the skin and the pressure-sensitive adhesive layer is absorbed by a deformation of the pressure-sensitive adhesive layer.

The adhesive patch of the invention can undergo a shear displacement of 18 $\mu$m to 1,000 $\mu$m at most, and preferably of 20 $\mu$m to 500 $\mu$m, without suffering a cohesive failure or interfacial separation.

By thus regulating the maximum shear displacement of the pressure-sensitive adhesive layer to a value within the range of 18 $\mu$m to 1,000 $\mu$m, not only skin irritation during wear can be relieved but also the adhesive patch is rendered less apt to partly or wholly peel off from the skin. In case where the maximum shear displacement of the pressure-sensitive adhesive layer is less than 18 $\mu$m, sufficient conformability to the skin is not obtained and microscopic separation and re-adhesion are thus apt to be repeated, resulting in physical irritation. On the other hand, in case where the maximum shear displacement of the pressure-sensitive adhesive layer exceeds 1,000 $\mu$m, a garment or the like is apt to adhere to the edges of the pressure-sensitive adhesive layer which have been exposed as a result of shear displacement, although this adhesive patch has satisfactory conformability to the skin. This adhesive patch hence tends to partly or wholly peel off from the skin. The interfacial separation of the pressure-sensitive adhesive layer suggests the possibility of partial or overall peeling of the adhesive patch. Further, in case where a cohesive failure has occurred, it becomes impossible to obtain the percentage recovery from shear displacement which will be described later. In addition, the cohesive failure is causative of the so-called "adhesive residue" after use of the adhesive patch.

In the invention, the maximum shear displacement of the pressure-sensitive adhesive layer of an adhesive patch is measured at 23°C in accordance with Procedure A as provided for in American Society for Testing Materials (ASTM) standard D3654. Incidentally, in the measurement, an adhesion area of (sample width)$\times$24 mm is employed, and a load is imposed so as to result in a shear stress of 1 N per 24 mm of the sample width. The shear displacement at the time when the deformation has finished is taken as the maximum shear displacement ($d_m$) of the pressure-sensitive adhesive layer.

The maximum shear displacement of a pressure-sensitive adhesive layer is considerably affected by the thickness of the pressure-sensitive adhesive layer. It is therefore possible to regulate the maximum shear displacement of a pressure-sensitive adhesive layer by regulating the thickness of the pressure-sensitive adhesive layer. The shear displacement (d) of a pressure-sensitive adhesive layer is generally expressed by the following equation (1).

$$d = H/(G \times \sigma) \qquad (1)$$

In equation (1), G is the modulus of rigidity of the pressure-sensitive adhesive layer, $\sigma$ is shear stress per unit area, and H is the thickness of the pressure-sensitive adhesive layer.

As shown by equation (1), the thicker the pressure-sensitive adhesive layer is, the larger the shear displacement of the pressure-sensitive adhesive layer is. Therefore, a larger thickness of the pressure-sensitive adhesive layer is advantageous from the standpoint of the ability to conform to large stretching/contraction of the skin. However, in case where the thickness of the pressure-sensitive adhesive layer is too large, since the exposed edges of the pressure-sensitive adhesive layer have a larger area, a garment or the like is apt to adhere to the pressure-sensitive adhesive layer when the adhesive patch is worn. As a result, such an adhesive patch is apt to partly or wholly peel off. Consequently, the thickness of the pressure-sensitive adhesive layer is preferably 70 μm to 300 μm, more preferably 80 μm to 250 μm.

The maximum shear displacement of a pressure-sensitive adhesive layer also is affected by the kinds and contents of ingredients contained in the pressure-sensitive adhesive layer. In addition, the maximum shear displacement thereof depends largely on the kind, molecular weight, and degree of crosslinking of the polymer constituting the pressure-sensitive adhesive layer. From the standpoint of regulating the maximum shear displacement of a pressure-sensitive adhesive layer to a value within the above-mentioned range, preferred polymers are acrylic polymers and rubber type polymers. Especially preferred are acrylic polymers. Furthermore, polymers having an absolute molecular weight of $1 \times 10^5$ to $6 \times 10^6$ are preferred, and polymers having an absolute molecular weight of $3 \times 10^5$ to $5 \times 10^6$, in particular, $1 \times 10^6$ to $4 \times 10^6$, are more preferred. In general, the maximum shear displacement of a pressure-sensitive adhesive layer tends to increase, for example, as the proportion by weight of the polymer contained in the pressure-sensitive adhesive layer decreases, although the maximum shear displacement thereof depends also on the kind of the polymer contained in the pressure-sensitive adhesive layer. Furthermore, there is a tendency that the higher the degree of crosslinking of the pressure-sensitive adhesive layer is, the smaller the maximum shear displacement thereof is.

<Percentage Recovery from Shear Displacement>

In order that an adhesive patch which has been applied to the skin and has deformed in response to a stretching/contraction movement of the skin may thereafter conform to the next stretching/contraction movement of the skin, the adhesive patch need to recover from the shear displacement without suffering a cohesive failure of the pressure-sensitive adhesive layer. Consequently, in the adhesive patch of the invention, the pressure-sensitive adhesive layer, upon release from a shear stress, has a percentage recovery from the shear displacement of preferably 85% or higher, more preferably 90% or higher. When the percentage recovery thereof from the shear displacement is 85% or higher, the effect of shear displacement can be maintained over a long period. On the other hand, even when the percentage recovery from the shear displacement is less than 85%, such a low percentage recovery exerts a slight influence when the adhesive patch is applied to a part where the skin is stretched and contracted not frequently. However, when this adhesive patch is applied to a part where the skin is repeatedly stretched and contracted, there is a possibility that the effect of relieving the stress due to shear deformation might be readily lost.

In the invention, the percentage recovery from shear displacement can be determined from the maximum shear displacement ($d_m$) of the pressure-sensitive adhesive layer and a residual shear displacement ($d_r$) remaining after release from the shear stress, using the following equation (2).

Percentage recovery from shear displacement (%)

$$= (d_m - d_r)/d_m \times 100 \qquad (2)$$

For heightening the percentage recovery from shear displacement, it is effective to crosslink the pressure-sensitive adhesive layer. It should, however, it should be noted that too high a degree of crosslinking results in a decrease in the maximum shear displacement of the pressure-sensitive adhesive layer. It is therefore required to crosslink the pressure-sensitive adhesive layer to a moderate degree, and the degree of crosslinking can be regulated by selecting the kind of crosslinking agent, amount of the crosslinking agent to be used, crosslinking conditions, etc., which will be described later.

<Pressure-Sensitive Adhesive Layer>

The pressure-sensitive adhesive layer can be formed by adding a polymer as a main ingredient to a solvent optionally together with other ingredients such as, for example, a drug, a tackifier, and an organic liquid ingredient, mixing the ingredients together to obtain a composition for pressure-sensitive adhesive layer formation, forming a layer of the composition on a release liner or a backing by a technique such as application, adhesion, fusion bonding, or melt bonding, and drying the layer of the composition. From the standpoint of adhesiveness to the skin, it is preferred that the pressure-sensitive adhesive layer be a hydrophobic pressure-sensitive adhesive layer. Consequently, a pressure-sensitive adhesive layer containing no water (non-hydroscopic pressure-sensitive adhesive layer) is preferred. From this standpoint, it is preferred that the solvent to be used for preparing the composition for pressure-sensitive adhesive layer formation be an organic solvent such as methanol, ethanol, benzene, n-hexane, ethyl acetate, or acetone. Hereinafter, unless otherwise indicated, the contents of the ingredients for constituting a pressure-sensitive adhesive layer are expressed in terms of % by weight based on the total weight of the composition for forming the pressure-sensitive adhesive layer excluding the solvent.

The polymer for constituting the pressure-sensitive adhesive layer is not particularly limited. Examples thereof include: acrylic polymers; styrene/diene/styrene block copolymers (e.g., styrene/isoprene/styrene block copolymers and styrene/butadiene/styrene block copolymers); rubber type polymers such as polyisoprene, polyisobutylene, and polybutadiene; silicone polymers such as silicone rubbers, dimethylpolysiloxane, and diphenylpolysiloxane; vinyl ether polymers such as poly(vinyl methyl ether), poly(vinyl ethyl ether), and poly(vinyl isobutyl ether); vinyl ester polymers such as vinyl acetate/ethylene copolymers; and polyester type polymers formed from a carboxylic acid ester ingredient, e.g., dimethyl terephthalate, dimethyl isophthalate, or dimethyl phthalate, and a polyhydric alcohol ingredient, e.g., ethylene glycol.

In the invention, from the standpoint of obtaining a pressure-sensitive adhesive layer in which (a) the logarithmic decrement of the surface of the pressure-sensitive adhesive layer, (b) the maximum shear displacement of the pressure-sensitive adhesive layer, and the percentage recovery from shear displacement of the pressure-sensitive adhesive layer are within the respective ranges shown above, it is preferred to use an acrylic polymer, a rubber type polymer, or the like as the polymer for constituting the pressure-sensitive adhesive layer. It is more preferred to use an acrylic polymer.

The acrylic polymer preferably is a polymer obtained by copolymerizing one or more functional monomers with one or more alkyl (meth)acrylic acid alkyl esters (alkyl (meth)acrylates) as a main monomer ingredient(s). Specifically, a copolymer formed from 60% to 99% by weight (preferably 65% to 95% by weight, based on total weight of all the monomers constituting the acrylic polymer) of one or more (meth)acrylic acid alkyl esters and one or more functional monomers as the remainder is preferred. The (meth)acrylic acid alkyl esters (hereinafter referred to also as "main monomer ingredient") for constituting the acrylic polymer usually are alkyl esters in which the alkyl groups each are a linear or branched alkyl group having 4 to 13 carbon atoms (e.g., butyl, pentyl, hexyl, heptyl, octyl, 2-ethylhexyl, nonyl, decyl, undecyl, dodecyl, or tridecyl). One or more members selected from these esters are used.

It is possible to use a (meth)acrylic acid alkyl ester having an alkyl group with 1 to 3 carbon atoms (e.g., methyl, ethyl, or isopropyl) and/or a (meth)acrylic acid alkyl ester having an alkyl group with 14 or more carbon atoms (e.g., tetradecyl pentadecyl, or hexadecyl) in combination with the (meth)acrylic acid alkyl esters having an alkyl group with 4 to 13 carbon atoms so long as the resultant acrylic polymer does not remain on the release liner or adherend. In this case, it is preferred that the content of the (meth)acrylic acid alkyl ester having an alkyl group with 1 to 3 carbon atoms and/or the (meth)acrylic acid alkyl ester having an alkyl group with 14 or more carbon atoms be 20% by weight or less based on the (meth)acrylic acid alkyl esters having an alkyl group with 4 to 13 carbon atoms.

The functional monomer to be copolymerized with the (meth)acrylic acid alkyl esters is a monomer which has in the molecule thereof at least one unsaturated double bond that takes part in the copolymerization reaction and which further has a functional group in a side chain thereof. Examples thereof include: carboxyl-containing monomers such as (meth)acrylic acid, itaconic acid, and maleic anhydride; hydroxy-containing monomers such as 1-hyrdoxyethyl (meth)acrylate, 2-hydroxyethyl (meth)acrylate, 2-hydroxypropyl (meth)acrylate, and 3-hydroxypropyl (meth)acrylate; amide-group-containing monomers such as (meth)acrylamide, N-methylol(meth)acrylamide, and N-(butoxymethy)(meth)acrylamide; amino-containing monomers such as 2-(diethylamino)ethyl (meth)acrylate and 2-(tert-butylamino)ethyl (meth)acrylate; sulfo-containing monomers such as 2-sulfoethyl (meth)acrylate; epoxy-containing monomers such as glycidyl (meth)acrylate; aziridine-group-containing monomers such as 2-(aziridin-1-yl)ethyl (meth)acrylate; allyl-containing monomers such as allyl (meth)acrylate; isocyanate-group-containing monomers such as 2-isocyanatoethyl (meth)acrylate; and alkoxy-containing monomers such as 2-methoxyethyl (meth)acrylate.

One or more of those functional monomers can be selected and used. From the standpoints of the pressure-sensitive adhesive properties and cohesiveness of the pressure-sensitive adhesive layer, the property of releasing the drug contained in the pressure-sensitive adhesive layer, etc., it is preferred to use carboxyl-containing monomers. Especially preferably, (meth)acrylic acid is used.

In the invention, a copolymer obtained by copolymerizing the (meth)acrylic acid alkyl ester(s) (main monomer ingredient) and functional monomer(s) together with one or more other monomers can be used as an acrylic polymer.

Examples of the other monomers include (meth)acrylonitrile, vinyl acetate, vinyl propionate, N-vinylpyrrolidone, N-vinylmethylpyrrolidone, 2-, 3-, or 4-vinylpyridine, N-vinylpiperidone, 4- or 5-vinylpyrimidine, vinylpiperazine, vinylpyrrole, 1- or 2-vinylimidazole, N-vinylcaprolactam, and 2- or 5-vinyloxazole. One or more members can be selected from these and used. The content of such other monomers is generally about 0% to 40% by weight, preferably about 10% to 30% by weight, based on the total weight of the (meth)acrylic acid alkyl esters (main monomer ingredient) and the functional monomers.

Preferred examples of the acrylic polymer to be used in the invention include a copolymer formed from 65% to 99% by weight of 2-ethylhexyl (meth)acrylate and 1% to 35% by weight of (meth)acrylic acid, and a copolymer formed from 60% to 99% by weight of 2-ethylhexyl (meth)acrylate and 1% to 40% by weight of N-vinylpyrrolidone.

The acrylic polymer may be incorporated into the pressure-sensitive adhesive layer in an amount of preferably 30% to 95% by weight, more preferably 30% to 90% by weight, as stated above.

In the invention, the pressure-sensitive adhesive layer may be a non-crosslinked pressure-sensitive adhesive layer, which had not undergone a crosslinking treatment. It is, however, preferred that the pressure-sensitive adhesive layer be a crosslinked pressure-sensitive adhesive layer, which has undergone a crosslinking treatment. Examples of techniques for the crosslinking include physical crosslinking treatments such as irradiation with light (ultraviolet) and irradiation with radiation (γ rays) and chemical crosslinking treatments such as use of a monomer for internal crosslinking and incorporation of an external crosslinking agent into the pressure-sensitive adhesive layer. From the standpoint of stability of the pressure-sensitive adhesive layer, chemical crosslinking treatments are preferred. Suitable external crosslinking agents include isocyanate compounds such as trifunctional isocyanates, organic peroxides such as diacyl peroxides (e.g., dibenzoyl peroxide), organometallic salts, metal alcoholates, metal chelate compounds (e.g., ethylacetoacetatoaluminum diisopropylate), and polyfunctional compounds (e.g., polyfunctional external crosslinking agents). Suitable monomers for internal crosslinking include polyfunctional monomers for internal crosslinking, such as diacrylates and dimethacrylates. Any of these crosslinking agents can be used for accomplishing the object of the invention, and a suitable crosslinking agent may be selected while taking account of the kind of the polymer, the kinds and contents of other components of the pressure-sensitive adhesive layer, e.g., an organic liquid ingredient, etc. In the case where a polymer obtained using a carboxyl-containing monomer, e.g., acrylic acid, as a comonomer is used, it is preferred to employ an isocyanate compound or a metal chelate compound. The amount of the external crosslinking agent to be used is preferably 0.05 parts to 5 parts by weight, more preferably 0.1 parts to 1 part by weight, per 100 parts by weight of the polymer contained in the pressure-sensitive adhesive layer, from the standpoint of the degree of crosslinking.

In the case where a chemical crosslinking treatment is to be conducted, it is preferred to subject the pressure-sensitive adhesive layer to a heat treatment in order to accelerate the crosslinking thereof. Examples of factors in conditions for the heat treatment include heating period and heating temperature. There is a tendency that the higher the heating temperature and the longer the heating period are, the lower the logarithmic decrement of the surface of the pressure-sensitive adhesive layer is. There are cases where the logarithmic decrement of the pressure-sensitive adhesive layer surface can be regulated also by regulating the humidity of the atmosphere for heating or by regulating the time period from formation of the pressure-sensitive adhesive layer to initiation of the heat treatment. Namely, although a pressure-sensitive adhesive layer may be rapidly subjected to a heat treatment after formation thereof, use may be made of a method in which the pressure-sensitive adhesive layer is stored under non-high temperature conditions, e.g., 0°C to 40°C, preferably 0°C to 30°C, for a certain period, e.g., 8 hours to 120 hours, preferably 12 hours to 96 hours, and then subjected to a heat treatment to thermally crosslink the pressure-sensitive adhesive layer.

This heat treatment can be conducted at a temperature of, for example, 50°C to 100°C, preferably 60°C to 80°C, for a period of, for example, about 3 hours to 120 hours, preferably about 6 hours to 96 hours, more preferably about 24 hours to 72 hours. It is preferred to conduct the heat treatment under low-oxygen conditions, for example, in an atmosphere having an oxygen concentration of 10% by volume or less, preferably 5% by volume or less.

In the case where a rubber type polymer is used as the polymer for constituting the pressure-sensitive adhesive layer or in other cases, a tackifier such as, for example, a rosin resin, polyterpene resin, coumarone-indene resin, petroleum resin, terpene-phenol resin, or xylene resin can be incorporated in order to impart moderate pressure-sensitive adhesive properties. One of such tackifiers may be used alone, or a mixture of two or more thereof may be used. Examples of the petroleum resin include aliphatic (C5-derived) petroleum resins, aromatic (C9-derived) petroleum resins, copolymer type (C5-C9-derived) petroleum resins, and alicyclic saturated hydrocarbon resins obtained by partly or wholly hydrogenating aromatic (C9-derived) petroleum resins. The alicyclic saturated hydrocarbon resins preferably are resins having a softening point as measured by the ring-and-ball method of 90°C to 150°C. In the case where one or more tackifiers are incorporated, a suitable range of the total content thereof is 10 parts to 100 parts by weight per 100 parts by weight of all polymers.

From the standpoint of accomplishing the object of the invention, it is preferred to incorporate an organic liquid ingredient into the pressure-sensitive adhesive layer. The organic liquid ingredient is not particularly limited so long as the ingredient is liquid at room temperature (25°C). In the case where a mixture of two or more organic liquid ingredients is used, these ingredients are not particularly limited so long as the final mixture is liquid at room temperature (25°C). Examples thereof include: higher alcohols such as oleyl alcohol and octyldodecanol; polyhydric alcohols such as glycerol, ethylene glycol, and polypropylene glycol; higher fatty acids such as caprylic acid and oleic acid; fatty acid esters such as isopropyl myristate, isopropyl palmitate, and ethyl oleate; esters of polybasic acids, such as diethyl sebacate and diisopropyl adipate; polyhydric alcohol/fatty acid esters such as diglycerol triisostearate, sorbitan monooleate, propylene glycol dicaprylate, polyethylene glycol monolaurate, and polyoxyethylene sorbitol tetraoleate; polyoxyethylene alkyl ethers such as polyoxyethylene lauryl ether; hydrocarbons such as squalane and liquid paraffin; vegetable oils such as olive oil and castor oil; silicone oils; pyrrolidone and derivatives thereof such as N-methylpyrrolidone and N-dodecylpyrrolidone; and sulfoxides such as decyl methyl sulfoxide. One of these substances can be used alone, or a mixture of two or more thereof can be used.

In the case of the patch preparation containing a drug, it is preferred to use a higher alcohol, a fatty acid ester, a polyhydric alcohol/fatty acid ester, or the like as the organic liquid ingredient, from the standpoint of improving drug penetration through the skin. From the standpoint of obtaining a pressure-sensitive adhesive layer in which the logarithmic decrement of the pressure-sensitive adhesive layer surface and the maximum shear displacement of the pressure-sensitive adhesive layer are within the respective ranges shown above, the organic liquid ingredient is incorporated in an amount of preferably 5% to 70% by weight, more preferably 10% to 70% by weight. As the content of the organic liquid ingredient increases, the logarithmic decrement of the pressure-sensitive adhesive layer surface tends to become higher and the maximum shear displacement of the pressure-sensitive adhesive layer tends to increase, although this tendency depends on the kind of the organic liquid ingredient.

<Backing>

It is preferred that the backing to be used in the adhesive patch of the invention be constituted of a material which is substantially impermeable to drugs and other substances, that is, a material which prevents the active ingredient, additives, and other components of the pressure-sensitive adhesive layer from passing through the backing and being lost through the back to cause a decrease in content.

In the invention, a film constituted of a resin alone or of a metal foil alone or a laminate of such films may be used as the backing. A laminate of a porous body with a resin film can be used in order to improve adhesiveness (anchoring effect) between the backing and the pressure-sensitive adhesive layer. In this case, a pressure-sensitive adhesive layer is formed on the porous-body-side of the laminate.

Examples of the porous body include porous films and sheets. When the term "sheet" means that the thickness is 200 $\mu$m or more, it is preferred to use a porous film. The porous film may be either a single-layer film or a multilayer film. Suitable is a porous film which produces an anchoring effect so as to inhibit the pressure-sensitive adhesive layer from moving relatively to the porous body. Specific examples thereof include paper, woven fabric, nonwoven fabric, knit fabric, films and metal foils which have been mechanically perforated, and laminates of these materials. Especially preferred of these, from the standpoints of handleability, etc., are paper, woven fabric, nonwoven fabric, and laminates of these. In particular, nonwoven fabric is preferred.

The porous body and the resin film may be made of the same material, or may differ in material. The porous object and the resin film can be laminated to each other by a known method. Various additives such as, for example, an antioxidant, a pigment, and an antistatic agent may have been incorporated into the porous object and the resin film unless the incorporation thereof impairs the features of the invention. The surfaces of the porous object and resin film may have undergone a treatment such as corona discharge treatment or ultraviolet irradiation treatment.

Examples of the materials of the porous body and resin film which constitute the backing include: polyester resins such as poly(ethylene terephthalate); polyamide resins such as nylons; olefin resins such as polyethylene and polypropylene; vinyl resins such as poly(vinylidene chloride) including Saran (registered trademark of Asahi Chemical Industry Co., Ltd. and The Dow Chemical Co., U.S.A.), poly(vinyl chloride), and ionomer resins including Surlyn (registered trademark of E.I. du Pont de Nemours & Co., Inc.); acrylic resins such as ethylene/ethyl acrylate copolymers; fluorocarbon resins such as polytetrafluoroethylene; and combinations of these.

Preferred examples of the materials of the porous body and resin film which constitute the backing are polyester resins and olefin resins such as polypropylene and polyethylene. Especially preferred are polyester resins, e.g., poly (ethylene terephthalate).

In the case where woven fabric or nonwoven fabric is used as the porous body, the basis weight thereof is preferably 5 g/m$^2$ to 50 g/m$^2$, more preferably 10 g/m$^2$ to 30 g/m$^2$, from the standpoints of ensuring a sufficient amount of interstices and producing an anchoring effect.

The resin film to be laminated to the porous body may be either a single-layer film or a multilayer film. It is preferred to use a film which is not porous and is made of a resin that is impermeable to active ingredients. This resin film functions to inhibit components of the pressure-sensitive adhesive layer from passing off through the back of the backing and thereby reducing the contents thereof. In addition, this resin film is advantageously used in order to produce the effect of so-called occlusive dressing technique (ODT) in the case where the pressure-sensitive adhesive layer contains a drug. The thickness of the resin film is preferably 0.5 $\mu$m to 10 $\mu$m, more preferably 1 $\mu$m to 7 $\mu$m.

Consequently, examples of preferred backings for use in the invention include a laminated film composed of a polyester resin film (more preferably, a poly(ethylene terephthalate) film) having a thickness of 0.5 $\mu$m to 10 $\mu$m, more preferably 1 $\mu$m to 7 $\mu$m and nonwoven fabric made of a polyester resin (more preferably, poly(ethylene terephthalate)) and having a basis weight of 5 g/m$^2$ to 50 g/m$^2$, preferably 10 g/m$^2$ to 30 g/m$^2$.

Incidentally, in the case where a nonstretchable resin film is used as a backing, it is difficult to relieve physical skin irritation during wear, as compared with the case in which a stretchable backing is used. Consequently, the invention is more advantageous because physical skin irritation can be relieved by regulating the material properties of the pressure-sensitive adhesive layer.

<Release Liner>

A release liner can be superposed on the pressure-sensitive adhesive surface of the pressure-sensitive adhesive layer of the adhesive patch in order to protect the pressure-sensitive adhesive surface until the adhesive patch is applied to the skin. The release liner is not particularly limited, and examples of the material thereof include materials which themselves are known in this field. Specifically, examples thereof include: films of polyester resins including poly(ethylene terephthalate); films of vinyl resins such as poly(vinyl chloride), poly(vinylidene chloride), and polystyrene; films of acrylic resins such as various acrylic and methacrylic polymers; polycarbonate resin films; polyimide resin films; films of cellulosic resins such as cellulose acetate, regenerated cellulose (cellophane), and celluloid; and laminated films which are laminates of wood-free paper, glassine paper, or the like with a polyolefin-based film. From the standpoints of safety, profitability, and prevention of drug transfer, it is preferred to use a polyester resin film among those films. The thickness of the release liner is generally 10 $\mu$m to 200 $\mu$m, preferably 25 $\mu$m to 100 $\mu$m.

The adhesive patch of the invention is useful as medical or hygienic materials for application to the skin, such as sticking plasters, tapes for the skin, and dressing materials for wound covering. The adhesive patch of the invention can be provided in the form of a film, sheet, pad, etc.

In the invention, drugs can be incorporated into the pressure-sensitive adhesive layer according to desires. Among adhesive patches of the invention, the patches in which the pressure-sensitive adhesive layer contains a drug are especially referred to as "patch preparations". The drugs used herein are not particularly limited, and either systemically acting drugs or locally acting drugs can be used. However, drugs which can be administered to a mammal such as a human being through the skin, i.e., which are percutaneously absorbable, are preferred. Examples of such drugs include adrenocorticosteroidal agents, nonsteroidal anti-inflammatory agents, antirheumatic agents, sleeping agents, antipsychotic agents, antidepressants, mood stabilizers, psychostimulant agents, anxiolytic agents, antiepileptic agents, therapeutic agents for migraine, anti-Parkinsonian agents, ameliorants for cerebral circulation/metabolism, anti-dementia agents, autonomic drugs, muscle relaxants, hypotensive agents, diuretics, hypoglycemic agents, antihyperlipidemic agents, antipodagrics, systemic anesthetics, local anesthetics, antibacterial agents, antifungal agents, antiviral agents, antiparasitic agents, vitamin agents, antianginal agents, vasodilators, antiarrhythmics, antihistaminic agents, mediator release inhibitors, leukotriene antagonists, female hormone drugs, thyroid hormone drugs, antithyroid agents, antiemetic agents, antidizzying agents, bronchodilators, antitussives, expectorants, and smoking renunciation aids. These drugs may be used in the form of a free substance or in the form of a salt.

The content of the drug in the patch preparation is not particularly limited so long as the effect of the drug for percutaneous absorption is sufficiently produced and the adhesive properties and other properties of the pressure-sensitive adhesive layer are not impaired. However, the drug is contained in the pressure-sensitive adhesive layer in an amount of preferably 0.01% to 60% by weight, more preferably 0.02% to 40% by weight. When the content thereof is less than 0.01% by weight, there are cases where the remedial effect is not sufficiently obtained. When the content thereof is more than 60% by weight, there is a possibility that skin irritation might occur. There are also cases where a further improvement in remedial effect is not obtained and such a high drug content is disadvantageous from the standpoint of profitability.

The patch preparation of the invention, which is a percutaneous absorption type preparation intended to act locally or systemically, can be provided as either a matrix type preparation or a reservoir type preparation. With respect to the shape thereof, the patch preparation can be of any of various shapes including a patch form, a pressure-sensitive adhesive tape form, and a sheet form.

<Processes for Production>

As described above, the adhesive patch of the invention is prepared by forming a pressure-sensitive adhesive layer on at least one surface of a backing using a composition for forming the pressure-sensitive adhesive layer. A release liner may be superposed on the pressure-sensitive adhesive layer formed on the backing. Alternatively, use may be made of a method in which a pressure-sensitive adhesive layer is formed on a release liner and a backing is then laminated onto the pressure-sensitive adhesive layer.

For industrially producing the adhesive patch of the invention, the following process, for example, is preferred from the standpoint of production efficiency.

First, a composition for forming the pressure-sensitive adhesive layer is applied to one surface of a release liner and dried to form a pressure-sensitive adhesive layer. A backing is superposed thereon to obtain a sheet from which an adhesive patch is to be die-cut. Alternatively, a composition for forming the pressure-sensitive adhesive layer is applied to at least one surface of a backing and dried to form a pressure-sensitive adhesive layer, and a release liner is superposed thereon to obtain a sheet from which an adhesive patch is to be die-cut. In the case where the pressure-sensitive adhesive layer contains a crosslinking agent, the sheet may be subjected to an aging treatment according to need. Thus, a crosslinked structure can be formed in the pressure-sensitive adhesive layer. The sheet which has undergone the crosslinking treatment is die-cut into a given size. Thus, an adhesive patch of the invention can be obtained.

It is preferred that the adhesive patch of the invention be put in a package and stored therein. The package can be produced from a packaging material in general use for the packaging of adhesive patches. Examples of the packaging material include: polyolefin resin films such as polyethylene films, polypropylene films, and polymethylpentene films; vinyl resin films such as poly(vinyl chloride) films, poly(vinylidene chloride ) films, poly(vinyl alcohol) films, polystyrene films, polyacrylonitrile films, and ionomer films; polyester resin films such as poly(ethylene terephthalate) films; polyamide resin films such as nylon films; cellulosic resin films such as cellophane; polycarbonate resin films; laminated films obtained by laminating these resin films; and laminated films obtained by laminating any of these resin films to aluminum. After the adhesive patch is put in a package produced from any of these packaging materials, the package can be sealed by a known technique, e.g., heat sealing.

The invention further provides a method for relieving skin irritation which occurs during wear of an adhesive patch. Specifically, the skin irritation occurring during wear of an adhesive patch can be relieved by regulating the material properties of the pressure-sensitive adhesive layer of the adhesive patch so that (a) the logarithmic decrement of the surface of the pressure-sensitive adhesive layer, as determined by the rigid-pendulum free-damped oscillation method, is in the range of 0.03 to 0.35 and (b) the maximum shear displacement of the pressure-sensitive adhesive layer, when a shear stress is imposed on the pressure-sensitive adhesive layer, is in the range of 18 $\mu$m to 1,000 $\mu$m, as described hereinabove.

Examples

The invention will be explained below in more detail by reference to Examples, but the invention should not be construed as being limited to the following Examples. The ratios, amounts in parts, and concentrations in % given in the following Examples and Comparative Examples are all by weight.

<Preparation of Adhesive Patches>

EXAMPLE 1

2-Ethylhexyl acrylate, N-vinylpyrrolidone, and acrylic acid were copolymerized in a ratio of 74/22/4 to prepare a polymer having an absolute molecular weight of $1.8 \times 10^6$. A hundred parts (solid amount) of this polymer, 100 parts of isopropyl myristate, and 0.35 parts of an isocyanate type crosslinking agent (trade name, "CORONATE HL"; manufactured by Nippon Polyurethane Co., Ltd.) were sufficiently mixed together with ethyl acetate while regulating the viscosity. Thus, a solution for pressure-sensitive adhesive layer formation was obtained. This solution for pressure-sensitive adhesive layer formation was applied, in such an amount as to result in a thickness of 100 $\mu$m after drying, to a release liner (thickness, 75 $\mu$m) which was made of poly(ethylene terephthalate) and had undergone a releasability-imparting treatment with a silicone. The solution applied was dried with heating to form a pressure-sensitive adhesive layer. Immediately thereafter, a backing was laminated to the pressure-sensitive adhesive layer so that the nonwoven fabric side of the backing was adhered to the pressure-sensitive adhesive layer. This laminate was put, within 8 hours of the laminating, in an environment replaced with dry nitrogen gas, and was heated therein at 70°C for 48 hours to obtain an adhesive patch of Example 1. The backing used was a laminate (overall thickness, about 35 $\mu$m) obtained by laminating a poly(ethylene terephthalate) film having a thickness of 2 $\mu$m to nonwoven fabric made of poly(ethylene terephthalate) and having a basis weight of 12 g/m$^2$.

EXAMPLE 2

An adhesive patch was prepared in the same manner as in Example 1, except that the period of the heating to be conducted after backing laminating was changed to 12 hours. Thus, the adhesive patch of Example 2 was obtained.

EXAMPLE 3

An adhesive patch was prepared in the same manner as in Example 1, except that the laminate obtained by laminating the backing was cold-stored at 5°C for 3 days in a dry nitrogen gas atmosphere before being heated at 70°C. Thus, the adhesive patch of Example 3 was obtained.

EXAMPLE 4

A solution for pressure-sensitive adhesive layer formation was prepared in the same manner as in Example 1, except that the crosslinking agent was replaced with 0.30 parts of ethylacetoacetatoaluminum diisopropylate, which is a metal chelate compound. An adhesive patch was prepared in the same manner as in Example 1, except that the solution was used to form a pressure-sensitive adhesive layer in a thickness of 80 $\mu$m. Thus, the adhesive patch of Example 4 was obtained.

EXAMPLE 5

An adhesive patch was prepared in the same manner as in Example 4, except that a pressure-sensitive adhesive layer was formed in a thickness of 200 $\mu$m. Thus, the adhesive patch of Example 5 was obtained.

EXAMPLE 6

An adhesive patch was prepared in the same manner as in Example 4, except that isopropyl myristate and ethylacetoacetatoaluminum diisopropylate were used in amounts of 186 parts and 0.35 parts, respectively. Thus, the adhesive patch of Example 6 was obtained.

EXAMPLE 7

A hundred parts (solid amount) of a commercial acrylate/vinyl acetate copolymer solution (trade name, "DURO-TAK 87-2196"; manufactured by Henkel Technologies Japan Ltd.), which contained a crosslinking agent, and 66.7 parts of oleyl alcohol were sufficiently mixed together with ethyl acetate while regulating the viscosity. Thus, a solution for pressure-sensitive adhesive layer formation was obtained. Using this solution, an adhesive patch in which the pressure-sensitive adhesive layer had a thickness of 100 $\mu$m was prepared in the same manner as in Example 1. Thus, the adhesive patch of Example 7 was obtained.

EXAMPLE 8

An adhesive patch was prepared in the same manner as in Example 7, except that 0.30 parts of ethylacetoacetatoaluminum diisopropylate was further added in preparing a solution for pressure-sensitive adhesive layer formation. Thus, the adhesive patch of Example 8 was obtained.

EXAMPLE 9

A hundred parts of a commercial polybutadiene rubber having a weight-average molecular weight of $4.6\times10^5$ and a cis content of 98%, 100 parts of an alicyclic saturated hydrocarbon resin (trade name, "ARKON P-100"; manufactured by Arakawa Chemical Industries, Ltd.), 86 parts of isopropyl myristate, and 0.6 parts of benzoyl peroxide were sufficiently mixed together with ethyl acetate while regulating the viscosity. Thus, a solution for pressure-sensitive adhesive layer formation was obtained. This solution for pressure-sensitive adhesive layer formation was applied, in such an amount as to result in a thickness of 100 $\mu$m after drying, to a release liner (thickness, 75 $\mu$m) which was made of poly(ethylene terephthalate) and had undergone a releasability-imparting treatment with a silicone. The solution applied was dried with heating to form a pressure-sensitive adhesive layer. Immediately thereafter, a backing was laminated to the pressure-sensitive adhesive layer so that the nonwoven fabric side of the backing was adhered to the pressure-sensitive adhesive layer. This laminate was put, within 8 hours of the laminating, in an environment replaced with dry nitrogen gas, and was heated therein at 80°C for 48 hours to obtain an adhesive patch of Example 9. The backing used was a laminate (overall thickness, about 40 $\mu$m) obtained by laminating a poly(ethylene terephthalate) film having a thickness of 3.5 $\mu$m to nonwoven fabric made of poly(ethylene terephthalate) and having a basis weight of 12 g/m$^2$.

COMPARATIVE EXAMPLE 1

An adhesive patch was prepared in the same manner as in Example 1, except that after the backing was laminated to the pressure-sensitive adhesive layer, the heat treatment was not conducted. Thus, the adhesive patch of Comparative Example 1 was obtained.

COMPARATIVE EXAMPLE 2

An adhesive patch was prepared in the same manner as in Example 4, except that a pressure-sensitive adhesive layer was formed in a thickness of 60 μm. Thus, the adhesive patch of Comparative Example 2 was obtained.

COMPARATIVE EXAMPLE 3

An adhesive patch was prepared in the same manner as in Example 4, except that isopropyl myristate was used in an amount of 11 parts. Thus, the adhesive patch of Comparative Example 3 was obtained.

COMPARATIVE EXAMPLE 4

An adhesive patch was prepared in the same manner as in Example 6, except that the ethylacetoacetatoaluminum diisopropylate was replaced with 0.35 parts of an isocyanate type crosslinking agent (trade name, "CORONATE HL"; manufactured by Nippon Polyurethane Co., Ltd.). Thus, the adhesive patch of Comparative Example 4 was obtained.

COMPARATIVE EXAMPLE 5

An adhesive patch was prepared in the same manner as in Example 9, except that benzoyl peroxide was used in an amount of 0.3 parts. Thus, the adhesive patch of Comparative Example 5 was obtained.

<Evaluation of the Adhesive Patches>

The adhesive patches of Examples 1 to 9 and Comparative Examples 1 to 5 were subjected to the following evaluation. The results thereof are shown in Table 1.

(1) Measurement of Logarithmic Decrement of Pressure-Sensitive Adhesive Layer Surface

RPT-3000W was used as a rigid-pendulum type viscoelastometer. Cylinder edge RBP-040, which was of the round bar type made of brass and having a diameter of 4 mm, and a rigid pendulum constituted of frame FRB-100 (moment of inertia, $6 \times 10^2$ g·cm$^2$; mass, 13 g) were used to make a measurement (all manufactured by A & D Company, Ltd.). With respect to each adhesive patch, samples having a width of 10 mm were examined while elevating the sample temperature from 23°C (room temperature) to 40°C.
In Table 1, values measured at 23°C are shown. In each of the adhesive patches of the Examples and Comparative Examples, the logarithmic decrement of the surface of the pressure-sensitive adhesive layer showed no clear change indicating a dependence on sample temperature. Consequently, it is thought that when the adhesive patch of the invention is worn on the skin, the temperature of the skin exerts only a small influence on that material property of the pressure-sensitive adhesive layer.

(2) Maximum Shear Displacement of Pressure-Sensitive Adhesive Layer

Each adhesive patch was examined in accordance with Procedure A as provided for in ASTM D3654 in the following manner. A sample having a width of 24 mm was applied to a stainless-steel plate so as to result in an adhesion area of 24 mm × 24 mm. While applying a shear stress of 1 N to this sample, an examination was made at 23°C. The shear displacement was measured with a digital microscope, and the shear displacement at the time when 30 minutes had passed was taken as the maximum shear displacement of the pressure-sensitive adhesive layer. In the case where the deformation continued even after 30 minutes had passed, it was deemed that a cohesive failure had occurred.

(3) Percentage Recovery from Shear Deformation

After the maximum shear displacement ($d_m$) of the pressure-sensitive adhesive layer was measured, the shear stress was removed. At 30 minutes thereafter, the shear displacement was measured with the digital microscope. This displacement was taken as the residual shear displacement ($d_r$), and the percentage recovery from shear displacement was calculated using the equation (2) given above.

(4) Skin Irritation during Wear and Adhesion to Skin

With respect to each adhesive patch, samples cut into a size of 10 cm$^2$ were used. The samples were worn

12

for 24 hours on an upper arm and the back (around a shoulder blade) of each of healthy volunteers as testers, and the testers evaluated skin irritation during wear and adhesion to the skin. However, the adhesive patches which suffered a cohesive failure in the measurement of the maximum shear displacement of the pressure-sensitive adhesive layer were not subjected to this evaluation.

With respect to skin irritation, senses including a pain, itch, uncomfortable feeling, and stretching feeling are inclusively taken as skin irritation. The testers evaluated these senses of skin irritation in accordance with the following 5-grade evaluation criteria. With respect to adhesion to the skin, the proportion of the adhesion area of each sample after the lapse of 24 hours to the adhesion area thereof at the initiation of wear was measured by eye, and the adhesion was evaluated in accordance with the following 5-grade evaluation criteria.

Evaluation criteria for skin irritation

No irritation: 4 points
Slight irritation: 3 points
Somewhat intense irritation: 2 points
Intense irritation: 1 point
Highly intense irritation: 0 point

Evaluation criteria for adhesion to skin

90% to 100%: 4 points
70% to less than 90%: 3 points
50% to less than 70%: 2 points
less than 50%: 1 point
Peeled off in 24 hours: 0 point

With respect to each adhesive patch, five samples were evaluated on each of the application parts, i.e., the upper arm and the back, and an average of the evaluation ratings was taken as the score of the samples. In the case where samples were applied to the same part of the same tester, the tests were conducted at an interval of 2 weeks or more.

(5) Adhesive Force

For reference, each adhesive patch was examined for adhesive force in accordance with Test Method A as provided for in ASTM D3330.
Table 1

| Sample | Logarithmic decrement | Maximum shear displacement [μm] | Percentage recovery from shear displacement [%] | Skin irritation | | Adhesion to skin | | Adhesive force [N/24 mm] |
|---|---|---|---|---|---|---|---|---|
| | | | | Upper arm | Back | Upper arm | Back | |
| Example 1 | 0.29 | 257 | 90 | 3.4 | 3.0 | 4.0 | 4.0 | 2.3 |
| Example 2 | 0.32 | 284 | 87 | 3.2 | 2.8 | 4.0 | 3.6 | 2.4 |
| Example 3 | 0.21 | 132 | 95 | 3.6 | 3.2 | 4.0 | 4.0 | 2.8 |
| Example 4 | 0.09 | 22 | 96 | 3.4 | 2.6 | 4.0 | 3.4 | 1.6 |
| Example 5 | 0.09 | 61 | 97 | 3.6 | 3.0 | 4.0 | 4.0 | 2.1 |
| Example 6 | 0.07 | 62 | 94 | 3.6 | 2.8 | 4.0 | 3.6 | 1.4 |
| Example 7 | 0.25 | 262 | 82 | 3.4 | 2.4 | 3.8 | 2.8 | 5.1 |
| Example 8 | 0.08 | 53 | 94 | 3.6 | 2.8 | 4.0 | 3.6 | 1.7 |
| Example 9 | 0.20 | 36 | 100 | 3.4 | 2.6 | 4.0 | 3.6 | 2.0 |
| Comparative Example 1 | 0.33 | cohesive failure | | | | | | |
| Comparative Example 2 | 0.09 | 15 | 93 | 2.8 | 1.6 | 3.8 | 2.2 | 2.0 |
| Comparative Example 3 | 0.17 | 5 | 100 | 2.2 | 1.2 | 3.6 | 2.0 | 6.3 |
| Comparative Example 4 | 0.43 | 816 | 83 | 2.6 | 1.6 | 3.6 | 2.0 | 1.7 |
| Comparative Example 5 | 0.54 | cohesive failure | | | | | | |

As shown in Table 1, the adhesive patches of the Examples, in each of which the logarithmic decrement of the pressure-sensitive adhesive layer surface and the maximum shear displacement of the pressure-sensitive adhesive layer were within the respective ranges according to the invention, were rated as substantially satisfactory with respect to both skin irritation during wear and adhesion to the skin.

More specifically, the adhesive patches of Examples 4 and 9 had a relatively small value of the maximum shear displacement of the pressure-sensitive adhesive layer, that is, the pressure-sensitive adhesive layers thereof were slightly less apt to deform. Because of this, these adhesive patches had a slightly low score with respect to skin irritation on the back, in which the skin is frequently stretched and contracted. However, the score of "2.6 points" on the back, where the skin stretching/contraction conditions are severe, is thought to be relatively satisfactory; the adhesive patches of Examples 4 and 9 were hence rated as sufficiently reduced in skin irritation during wear. The adhesive patches of the Examples except Example 7 had a recovery from shear displacement of 85% or higher and showed satisfactory adhesion to the skin in application to both the upper arm and the back. The adhesive patches of Examples 1 and 3, in each of which the pressure-sensitive adhesive layer had a large value of maximum shear displacement and a high percentage recovery from shear displacement, were low in skin irritation on each of the upper arm and the back. The pressure-sensitive adhesive layers in these adhesive patches satisfactorily recover from the shear displacement which accompanies skin stretching or contraction, and these adhesive patches are hence thought to be sufficiently conformable to the skin.

On the other hand, the adhesive patches of Comparative Examples 2 and 3, in each of which a value of the maximum shear displacement of the pressure-sensitive adhesive layer is small but the logarithmic decrement of the pressure-sensitive adhesive layer surface was within the range specified in the invention, and the adhesive patch of Comparative Example 4, in which the logarithmic decrement of the pressure-sensitive adhesive layer surface was high but the maximum shear displacement of the pressure-sensitive adhesive layer was within the range specified in the invention, aroused clear skin irritation when worn on the skin. These adhesive patches were rated as low also in adhesion to the skin in the back. Furthermore, the adhesive patch of Comparative Example 1, which had been prepared without conducting a heat treatment after laminating of the backing to the pressure-sensitive adhesive layer, and the adhesive patch of Comparative Example 5, for which a lower crosslinking-agent content than for Example 9 had been used, suffered a cohesive failure due to the shear force.

The results of the evaluation of the adhesive patches of Examples 1 to 3 and Comparative Example 1 suggest that the maximum shear displacement of the pressure-sensitive adhesive layer of an adhesive patch and the percentage recovery from shear displacement of the pressure-sensitive adhesive layer are considerably affected by the conditions under which the patch was produced. Namely, the results indicate the possibility that those material properties might be regulated by conducting a heat treatment, i.e., thermal crosslinking, after laminating a backing to a pressure-sensitive adhesive layer when an adhesive patch is produced. The results of the evaluation of the adhesive patches of Examples 4 and 5 and Comparative Example 2 suggest that the maximum shear displacement of a pressure-sensitive adhesive layer can be regulated without affecting the logarithmic decrement of the surface of the pressure-sensitive adhesive layer, by regulating the thickness of the pressure-sensitive adhesive layer. Furthermore, the results of the evaluation of the adhesive patches of Example 4 and Comparative Example 3 suggest that the ratio of the polymer content to the content of an organic liquid ingredient in the pressure-sensitive adhesive layer affects those material properties. The results of the evaluation of the adhesive patches of Example 6 and Comparative Example 4 suggest that the kind of crosslinking agent affects those material properties. The results of the evaluation of the adhesive patches of Example 9 and Comparative Example 5 suggest that the content of a crosslinking agent affects those material properties.

Incidentally, the evaluation results given in Table 1 showed no correlation between the adhesive force of an adhesive patch and either skin irritation during wear or adhesion to the skin. Consequently, it is suggested that skin irritation at the time of peeling, which is attributable to the adhesive force of the adhesive patch, is unrelated to skin irritation during wear.

<Production of Patch Preparations>

EXAMPLE 10

To 19 parts of diphenhydramine hydrochloride was added an equimolar amount of sodium hydroxide in the form of ethanol solution. The ingredients were sufficiently mixed to prepare a drug solution. To this drug solution were added 100 parts of the same polymer as used in Example 1, 119 parts of isopropyl myristate, and 0.38 parts of ethylacetoacetatoaluminum diisopropylate. The ingredients were sufficiently mixed while regulating the viscosity by further adding ethyl acetate thereto. Thus, a solution for pressure-sensitive adhesive layer formation was obtained. This solution for pressure-sensitive adhesive layer formation was applied, in such an amount as to result in a thickness of 200 $\mu$m after drying, to a release liner (thickness, 75 $\mu$m) which was made of poly(ethylene terephthalate) and had undergone a releasability-imparting treatment with a silicone. The solution applied was dried to form a pressure-sensitive adhesive layer. Immediately thereafter, a backing was laminated to the pressure-sensitive adhesive layer so that the nonwoven fabric of the backing was adhered to the pressure-sensitive adhesive layer. This laminate was put, within 8 hours of the laminating, in an environment replaced with dry nitrogen gas, and was heated therein at 70°C for 48 hours to obtain a patch preparation of Example 10. The backing used was a laminate (overall thickness, about 35 $\mu$m) obtained by laminating a poly(ethylene terephthalate) film having a thickness of 2 $\mu$m to nonwoven fabric made of poly(ethylene terephthalate) and having a basis weight of 12 g/m$^2$.

EXAMPLE 11

The release liner of the adhesive patch of Example 9 was stripped off, and a hexane solution of propranolol was applied to and infiltrated into the exposed surface of the pressure-sensitive adhesive layer so as to result in a propranolol content of 1.4% after drying. This pressure-sensitive adhesive layer was dried, and the release liner was then superposed thereon again. Thus, a patch preparation of Example 11 was obtained.

<Evaluation of the Patch Preparations>

The patch preparations of Examples 10 and 11 were examined for the logarithmic decrement of the pressure-sensitive adhesive layer surface, maximum shear displacement of the pressure-sensitive adhesive layer, and percentage recovery from shear displacement in the same manners as for the adhesive patches of Examples 1 to 9 and Comparative Examples 1 to 5. The results thereof are shown in Table 2.

Table 2

| Sample | Logarithmic decrement | Maximum shear displacement [$\mu$m] | Percentage recovery from shear displacement [%] |
|---|---|---|---|
| Example 10 | 0.10 | 57 | 100 |
| Example 11 | 0.21 | 38 | 95 |

In the patch preparations of Examples 10 and 11, the values of the logarithmic decrement of the pressure-sensitive adhesive layer surface, maximum shear displacement of the pressure-sensitive adhesive layer, and percentage recovery from shear displacement were within the respective preferred numerical ranges according to the invention. Consequently, it is deemed that the patch preparations of Examples 10 and 11 are low in skin irritation during wear and have satisfactory adhesiveness to the skin.

As described above in detail, an adhesive patch and a patch preparation which are reduced in skin irritation during wear, in particular, skin irritation in parts of the body where the skin is frequently stretched and contracted, and which are less apt to partly or wholly peel off the skin can be provided according to the invention.

While the present invention has been described in detail and with reference to specific embodiments thereof, it will be apparent to one skilled in the art that various changes and modifications can be made therein without departing from the scope thereof.

This application is based on Japanese patent application No. 2010-169751 filed July 28, 2010, the entire contents thereof being hereby incorporated by reference.

**Claims**

1.  An adhesive patch comprising a backing and a pressure-sensitive adhesive layer formed on at least one surface of the backing,
    wherein (a) a surface of the pressure-sensitive adhesive layer has a logarithmic decrement, as determined by the rigid-pendulum free-damped oscillation method, in the range of 0.03 to 0.35, and
    (b) the pressure-sensitive adhesive layer has a maximum shear displacement in the range of 18 $\mu$m to 1,000 $\mu$m.

2.  The adhesive patch according to claim 1, wherein the pressure-sensitive adhesive layer, upon release from the shear stress, has a percentage recovery from the shear displacement of 85% or higher.

3.  The adhesive patch according to claim 1, wherein the pressure-sensitive adhesive layer is a crosslinked pressure-sensitive adhesive layer comprising a polymer and an organic liquid ingredient.

4.  The adhesive patch according to claim 2, wherein the pressure-sensitive adhesive layer is a crosslinked pressure-sensitive adhesive layer comprising a polymer and an organic liquid ingredient.

5.  The adhesive patch according to claim 3, wherein the pressure-sensitive adhesive layer has been crosslinked with an external crosslinking agent.

6.  The adhesive patch according to claim 4, wherein the pressure-sensitive adhesive layer has been crosslinked with an external crosslinking agent.

7.  A patch preparation, which is the adhesive patch according to any one of claims 1 to 6, wherein the pressure-sensitive adhesive layer further contains a drug.

**EP 2 412 390 A2**

**REFERENCES CITED IN THE DESCRIPTION**

**Patent documents cited in the description**

- JP 5065460 A
- JP 5139960 A
- JP 6319792 A

- JP 6343685 A
- JP 2010169751 A